# EUROPEAN PATENT APPLICATION

(11) **EP 1 447 048 A1**
(43) Date of publication of application: **18.08.2004**
(21) Application number: 04075890.6
(22) Date of filing: 14.11.1995
(51) Int. Cl.: A61B 8/12, A61M 25/00

(54) **Device for examinating and measuring cavities by means of acoustic reflectometry.**

(30) Priority: 15.11.1994 DK 130494
(62) Divisional of application: 95936990.1
(71) Applicant: RhinoMetrics A/S, 3540 Lynge (DK)
(72) Inventor: Rasmussen, Steen Barbrand, 3540 Lynge (DK)
(74) Representative: Jorgensen, Bjorn Barker

(57) **Abstract**

For reflectometric examination and measurement of human or animal cavities, e.g. examination in air passages, eating passages, etc., the device includes a flexible hose (1), which via the passage in question is introduced forward to the cavity in question, with the distal end of the hose placed past the zone of that passage which is to be examined.

A transducer (3) converts an activation signal from a signal source (2) into an excitation signal which is sent forward in the interior of the hose. A response signal dependant on local deformations of the hose at the examined zone is picked up by a transducer (5) and subjected to analysis relative to the excitation signal. An analysis circuit (4) and a computer (6) submit on a screen (7) an image indicating the results of the examination.

## Description

The present invention relates to a device for reflectometric examination and gauging of cavities, and of the type comprising an acoustic signal source, a hose with a distal end to be introduced via an entrance to the cavity to be examined, at least a first transducer connected to the signal source for transfer of an activating signal originating from the signal source to the hose, at least a second transducer connected to the hose for reception of response signals from the cavity, and a computer adapted for analysis of the response signal in relation to the excitation signal transmitted to the cavity.

For examination and measurement of blockings, deformations, movements, etc. in various cavities in humans and animals, e.g. pharynx, larynx and the other air passages, alimentary passages, blood vessels, etc. various methods are known.

In e.g. catheter examinations, balloon-angioplasty, etc., it is known to use a probe in the shape of a hose of flexible material.

Another method is based on measurement of reflection (reflectometry) using an acoustic, transient excitation signal, which through a tube and through the patient's mouth is sent into the air passages of the patient, cf. e.g. US-A-4.326.416.

Yet another method based on the use of a non-transient excitation signal - a random or pseudorandom signal - is used in pieces of equipment manufactured by the applicant company under the brand name SRE 2000 and SRE 2000 PC.

Pressure transducers placed in or on catheters to be introduced via the nose or the mouth have mainly been in use so far, especially in connection with examination of movements in the air passages and examination of stertorous respiration. This allows for measuring pressure variations, contractions, etc. in nose and throat.

A drawback of this technique is to be seen in that the measuring probe must comprise a relatively large number of closely spaced pressure transducers connected to a matching apparatus by means of which the position of and the pressure at each particular, examined spot may be determined with a sufficient resolution on a screen.

The state of the art also includes endoscopy, in which examination is made optically of nose, pharynx and other internal organs. These examinations have, however, a number of limitations, including the clarity and size of the optical image, the size of the catheter and in particular, lack of catheter flexibility which renders the catheter unsuitable for examination of e.g. stertorous respiration.

CT and MRI scanning have been tried, but involve long measuring time, which do not give useful measurements and not dynamic measurements at all.

By using acoustic reflectometry of the above-mentioned type, it is known that it is possible to measure cross-sectional areas in the air passages as a function of the distance from the transducer used for emitting the excitation signal, cf. the above-mentioned US-A-4.326.416 or an article: "Airway geometry by analysis of acoustic pulse response measurements" by Andrew C. Jackson et al. in J. Appl. Physiology, 43(3): 525-536, 1977.

In direct measuring, the measurements are limited by cross modes, i.e. cross resonances, and by adjacent cavities, which, to a large extent, limits the use of such direct measurements, if there are large differences in the cross-sectional areas as a function of the distance from the signal source.

In particular, when examining stertorous respiration, the use of direct measurements is impeded by the transient or continuous sound action necessary for the measurements, which affects the sleep state or wakes up the patient during the very examination phase, and causes measurement errors as well because of noise reaching the measuring microphone and a measurement error due to the very large cavity made up by the mouth and pharynx.

The invention aims at remedying the above mentioned disadvantages and to this end, a device of the type mentioned at the outset is characteristic according to the invention in that the hose is adapted to be introduced, seen from the signal-emitting transducer, forward to the cavity in question, with its distal end placed past the zone to be examined; that it consists of a thin-walled, soft, elastic plastics or elastomeric material; and that it is equipped at its proximal end with said at least one transducer, connected to said signal source, said transducer being adapted to transform an activation signal originating from the signal source into an excitation signal which is able to propagate through the hose and give rise to a response signal, caused by any deformation of the wall of the hose, back to the transducer.

The invention is based on the recognition that humans have many spots where an acquired or pathological change or obstruction in e.g. the air passages, urinary passages, etc. may be difficult to determine, locate and gauge with the above mentioned, state-of-the-art technique, i.a. because of cross resonances occurring in the large or small surrounding cavities to be found at the spot which is to be examined, and that it is particularly appropriate to use a hose having very flexible walls, which can be made to abut the side wall of the passage in question or can be deformed by some change, e.g. a contraction, at the spot in question. By this, the measuring equipment is brought to only "seeing", so to speak, the interior of the hose and clearly and accurately measuring the least deformation in the inner cross-sectional area of the hose as a function of the distance from the spot in the hose where the excitation signal is emitted and forward to the spot or spots where a deformation or deformations appear and from where the response signal originates.

According to a particularly appropriate embodiment of the invention, the device may be characteristic in that the hose has a longitudinal, axially centred lumen surrounded by a ring-shaped wall, and around the ring-shaped wall a number of spaced, longitudinal canals separated from each other by means of essentially radial partitions.

By combining reflectometric measurements made inside the lumen of the hose and the peripheral canals or chambers, it is possible with much greater sensibility to determine the position or gauge the areas in the portions of the hose which due to some local change or obstruction in the passage in question (air passage, urinary passage, etc.) are compressed, as well as the degree of compression.

Upon insertion of the hose it is possible, with or without action of positive or negative pressure in the lumen of the hose and/or the canals, by measuring the inner cross-sectional areas of the hose as a function of the distance, to determine the areas, which are most narrow or which compress the hose locally.

It is thus possible, in e.g. balloon examinations and widening of blood vessels in case of arteriosclerosis, to check the distension (cross-sectional area per unit longitudinal distance) of the balloon at the end of a catheter concurrently with the inflation of the balloon.

The invention is explained in more detail below, with reference to the schematic drawings, in which:
Fig. 1 shows a block diagram of the basic layout of the device according to the invention,
Fig. 2 is a perspective view of a portion of the hose according to the invention, at the spot of measuring,
Fig. 3 is a perspective view of a portion of the hose in another embodiment of the invention,
Fig. 4 is a sectional view of the hose in Fig. 3 in a sectional plane orthogonal to the axis of the hose,
Fig. 5 illustrates the placing of a hose according to the invention in the upper air passages with a patient, for the examination of tongue fallback,
Fig. 6 illustrates the placing of a hose according to the invention in the upper air passages with a patient, for the examination of stertorous respiration, and
Fig. 7 shows a schematic view of a hose with a balloon for a catheter examination in a blood vessel.

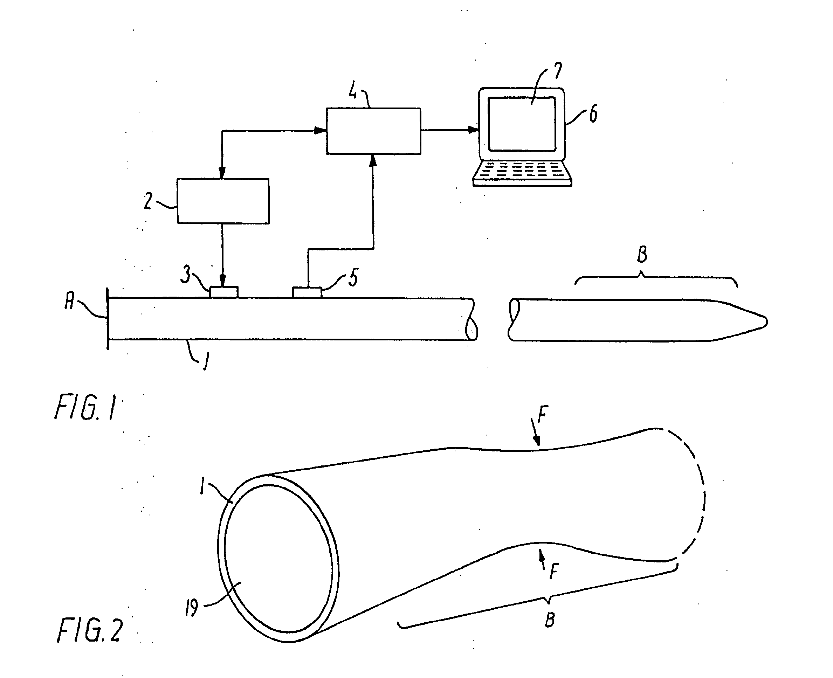

Fig. 1 shows the basic layout of the device according to the invention.

At 1, a hose is shown, the design of which will be explained below. At its proximal end A, the hose 1 is connectible in a manner known per se, not shown, to auxiliary equipment used for inserting the hose into e.g. the air passages of a patient, e.g. through the mouth or the nostrils, or into the urinary passages or a blood vessel. At B is shown the distal end of the hose which after insertion of the hose will be situated in the cavity of the patient who is to be subjected to examination.

At 2 is shown an electronic signal source, adapted to submit an activation signal to a transducer 3 which is connected to the hose 1. The signal source 2 submits the same signal to a signal analysis processor 4. At 5 is shown a transducer connected to the hose 1. When an excitation signal is transferred from the signal generator 2, via the transducer 3, to the interior of the hose 1, this signal will propagate in the hose, forward to the distal end of the hose, from where a response signal is sent back and received by the transducer 5 and from there carried to the signal analysis processor 4.

The signal analysis processor 4 is connected to a computer 6 by means of which it is possible, on an image screen 7 to present an image which illustrates the results of the examination and gauging made.

The transducer 3 can be of an arbitrary type known *per se,* e.g. an electromagnetic transducer, an electrostatic transducer, a piezo-electric transducer, etc. Its task is to transform the electronic signal from the signal source 2 into an excitation signal in the interior of the hose 1.

The transducer 5 may likewise be of an arbitrary, above-mentioned type, e.g. a microphone, the purpose of which is to receive the acoustic response signal from the distal end of the hose and to transform this response signal into an electric signal which is carried to the signal analysis processor 4.

The excitation signal may be a transient signal in the low frequency band, as known from e.g. the above US-A-4.326.416 or from the Jackson article. It may also be a non-transient excitation signal - a random or pseudo-random signal - as used in the above-mentioned pieces of equipment SRE 2000 and SRE 2000 PC.

The invention is a very important contribution to determining the exact position of the obstruction and to measure when and for how long the obstruction is lasting. It will thus be possible to connect an alarm system to the measuring equipment which gives an alarm when the probe has been compressed for a certain fixed period of time.

The analysis itself of the response signal in relation to the excitation signal belongs to a technique known *per se.*

Fig. 2 shows a portion of the hose 1 in the zone B of the hose. The characteristic feature of the hose according to the invention is that it is thin-walled, at least in its zone B at the distal end. The hose according to Fig. 2 is a simple hose, i.e. a hose with only one lumen 19.

If the hose 1, as will be explained later, is exposed locally, i.e. in the zone B mentioned, to an external mechanical influence (as implied by the arrow F), due to e.g. a contraction of the air passage, the oesophagus or a blood vessel of the patient, the resulting reduction of the diameter of the hose in said zone B will bring about a modification of the response signal, a modification which is to be seen in the image analysis and on the image screen. This modification expresses the change that might be present in the patient, e.g. a contraction.

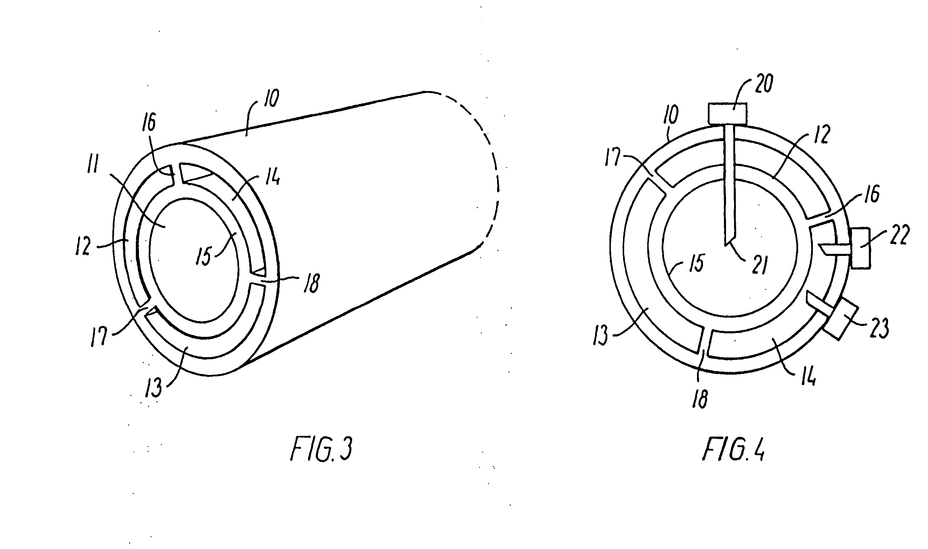

Fig. 3 shows a further developed embodiment of a hose according to the invention. The hose 10 has a central lumen 11 and three peripheral, annular-segment shaped canals or chambers 12, 13, 14. Such a hose may e.g. be made by extrusion of a soft plastics material or an elastomer. The outer diameter of the hose may amount to between e.g. 1 mm and e.g. 3-4 mm, depending on the intended use. The wall 15 around the central lumen 11 is continuous in the longitudinal direction of the hose and it separates the lumen 11 from the three peripheral chambers 12, 13, 14. The chambers themselves which are also continuous in the longitudinal direction of the hose, are separated from each other by means of radial partitions 16, 17, 18.

Fig. 4 shows a cross-sectional view of the hose in a plane orthogonal to the axis of the hose.

A transducer 20 has been put from the outside through e.g. the outer chamber 12 and through the wall 15 in such a way that the response-signal-receiving end 21 of the transducer 20 is located within the lumen 11.

Fig. 4 also shows two transducers 22, 23, which have been put from the outside into the outer wall of the hose 11 and whose response-signal-receiving ends 24, 25, respectively, are located in a peripheral chamber, e.g. chamber 14.

While the sectional view in Fig. 4 shows the two transducers situated in the sectional plane (the plane of the paper) it must be understood that they need not be and that e.g. transducer 23 may well be placed axially displaced from the transducer 22.

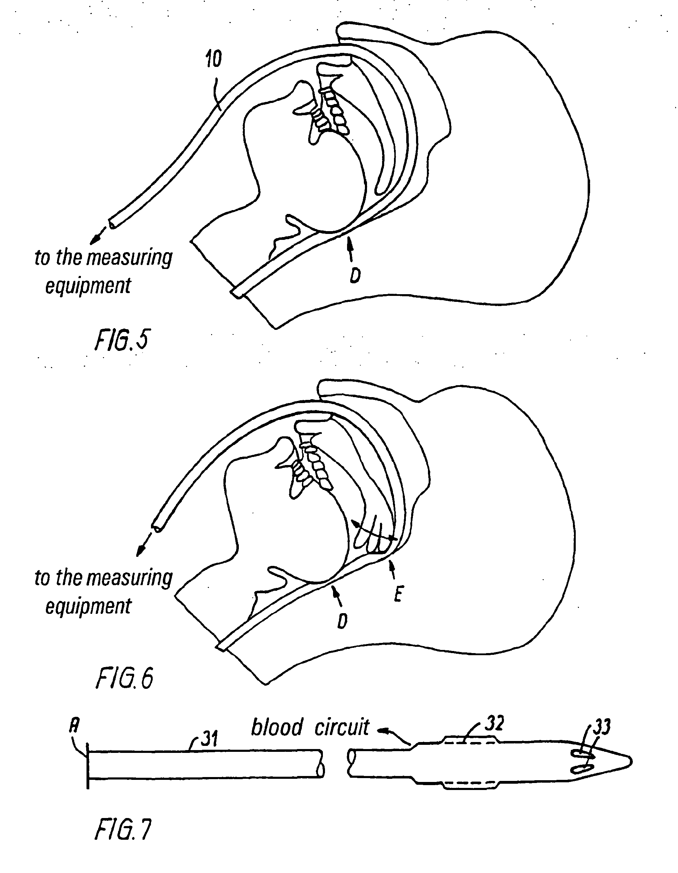

Fig. 5 illustrates the use of the hose for determining the position of and measuring the so-called tongue fallback with a patient, i.e. the situation where the patient's tongue constricts the upper air passages.

Here, the hose has been introduced through the nostrils and into the air passage. A portion of the hose has been compressed by the rear end of the tongue in the zone D.

Fig. 6 illustrates the use of the hose in order to determine with a patient the position of and measure the outbreak of vibrations in the soft palate (velum palatum) .

Fig. 6 shows the situation illustrated in Fig. 5 as well as the situation where said soft parts of the palate compress the hose in the zone E.

The principle of operation of the device according to the invention will now be explained.

It should be recalled, as mentioned in the preamble of the specification, that it is possible with the measuring approach known from US-A-4.326.416 and from the Jackson article as well as that used in the known measuring equipment of the applicant company, to determine the cross-sectional area of a cavity as a function of the distance from the excitation-signal-emitting transducer to the spot of measurement.

While the state of the art has the disadvantage that the measurements may be disturbed by cross modes (i.e. cross resonances) which is e.g. the case in examination of the air passages and the lungs with a patient, the approach according to the invention has the essential advantage that it is the inner cavity of the hose which constitutes the measurement cavity proper, which will be modified when needed by e.g. a contraction of the passage in which the hose has been introduced. The construction of the hose excludes the outbreak of cross resonances as in the state of the art. If the hose, which as mentioned has thin and flexible walls, is affected locally by a contraction, one or more of the outer chambers 12, 13, 14 and/or the central canal (lumen 19, Fig. 2, or lumen 11, Fig. 3) is affected mechanically by this contraction, which is immediately being gauged by the measuring equipment.

Supposing that the hose has the design shown in Figs 3 and 4 and that it has been introduced into the air passage with the patient as shown in Fig. 5. The mechanical compressive action from e.g. the rear end of the tongue onto the hose may e.g. influence only one of the outer chambers, e.g. the outer chamber 14, which may be detected electronically in the measuring equipment, or possibly the second and third outer chamber as well.

The invention thus offers the possibility for so to speak "differentiated" determination of position and measurement of the cross sectional area in the zone in question, as a function of the distance from the excitation-signal-emitting transducer in question to the zone in question.

If it is e.g. only the outer chamber 14, as stated in the previous paragraph, which in a patient's air passage is influenced by e.g. the rear portion of the tongue, it will solely be the transducer(s) belonging to the chamber 14 that will react.

As mentioned, Fig. 6 illustrates the situation where a patient is to be examined for vibrations in the soft parts of the palate, i.e. typically stertorous respiration. The vibrations in the zone E will influence at least one of the outer chambers of the hose and the measurement equipment will then be able to carry out the determination of position and the measurement.

Another field of particular medical and surgical interest for the invention is examinations of constrictions, e.g. calcification or other pathologic disorder in blood vessels, e.g. at the heart.

Fig. 7 shows another embodiment of the hose according to the invention, intended for this kind of examination.

The distal end of the hose 31 is in a manner known *per se* (normal catheter technique for widening blood vessels) designed with an inflatable balloon 32. It may be inflated by supply of pressure through longitudinal canals (not shown) in the outer wall of the hose. Between the balloon 32 and the distal end of the hose there is, in a manner known *per se,* a number of openings 33 for ensuring blood passage and at some distance away from the balloon 32 in the direction towards the proximal end of the hose there are outlets (not shown) for the circulating blood.

In the case where it is medically or surgically advisable to disrupt temporarily the blood circulation through the hose in order to perform gauging and/or widening, it will be possible to use a hose which does not have a canal for circulating blood, i.e. having neither openings 33 nor matching outlets.

Upon introduction of the hose it is possible, in the above-mentioned way, to perform detection of position and gauging of the constriction or the calcification, as well as any widening.

It will be within the scope of the invention to design the hose without the above-mentioned balloon, and instead manufacture the hose so that it has at its distal end, i.e. where the balloon otherwise would be placed, a considerably thinner and/or considerably more flexible outer wall. It will be within the scope of the invention as well to design the hose near its proximal end with means (not shown) for establishing a negative or positive pressure, e.g. with a liquid, in the lumen and/or in each chamber. Such a positive pressure will bring about a distension of said thinner and/or more flexible part of the hose at the distal end. Whether the hose has a balloon or not, whether it is distended or not and whether one or more chambers are pressed inwards by the constriction in the blood vessel, the measuring equipment will create a picture of the conditions in the area in question.

A hose has been described above with one single lumen or with one central lumen and peripheral chambers, but it will be within the scope of the invention to design a hose with two axial canals or a central lumen and two, four or e.g. five peripheral chambers.

Obviously, medical or surgical considerations decide the choice of the inner and outer dimensions of the hose, which is why the hose may be manufactured in different sizes (including the length), while the measuring equipment decides the upper frequency limit if a transient signal is used, as well as the other physical parameters.

If the hose according to the invention is to be used for examination of the breathing organs, the necessary supply of air or other gas to the patient may take place through the inlet of the hose (A in Fig. 1) and through a canal to the openings 33 (Fig. 7) at the distal end of the hose. In that case, the response signal originating from e.g. one or more of the peripheral chambers, may be separated electronically in the measuring equipment from the response signal coming from the lungs, due to the difference in signal transit time.

A particular example of the use of the invention has already been mentioned.

It is in the nature of things that exact examinations of persons who have their air passages blocked during sleep, and who are also described as snorers, are very difficult, and through the ages many failed corrective operations have been made on these patients.

The invention is a very important contribution to the determination the exact position of the blocking and to the gauging of when and for how long the blocking lasts. It will thus be possible to connect an alarm system to the measuring equipment, giving an alarm when the probe has been compressed for a certain, fixed period of time.

To-day, a pulsoxymeter (an instrument measuring the concentration of oxygen in the blood) will be connected during these examinations. An alarm is therefore activated when the concentration reaches certain predetermined limits.

It is not the stertorous respiration itself which is a risk, but the period of time during which the patient does not breathe because of a blocking.

This is the reason why equipment which acoustically registers the stertorous respiration will not be able to activate an alarm with sufficient certainty, as the non-occurrence of the "snoring sound" may either be due to a quiet, steady respiration with a low, regular flow, which is all right, or to the air passages being blocked for a long time. The hazard resides here.

In order to stress the importance of the invention it should be mentioned that the lack of oxygen to the lungs for such a long time considerably increases the risk of brain damages and thrombosis, in particular for older overweight persons.

By gauging internally, the advantage is achieved that the patient will not be awakened during the measuring by the excitation signal and at the same time, the measurements are not influenced to any significant extent by the high-tone sound spectrum of the snoring sounds.

The measurement probe itself is thus very easily introduced ambulatory into the patient before the night, typically through the nose, in cooperation with a doctor or a nurse.

A correct "tightening" through the nose takes place automatically due to the reflectory swallowing, and a connection (transducer, microphone part) at the end which projects out from the nose may be made without problems.

A synchronisation of the area measurements with the snoring sound may easily be made, either by means of an external microphone, e.g. one of the transducers 22, 23 (Fig. 4) or by using portions of the low frequency signal received by the measuring microphone.

It should be noted as well that the measuring equipment (hardware, software) which adequately performs the measurements in the separate chambers and during the measuring changes the static pressure in the separate chambers, may also concurrently provide information about the elasticity of the tissue giving counter-pressure to the surface of the chambers.

By establishing a pressure in the hose and a concurrent supply of acoustic energy in the infrasound interval up to 200 Hz internally in the lumen and the chambers, and a synchronisation of this infrasound signal with the acoustic-rhinometric (reflectometric) measurements, valuable information will thus be obtained on the elasticity in the walls to which the hose wall will contact during the various loading (pressure) conditions.

Considering that these types of transducer, e.g. a piezoelectric transducer, functions in both directions, i.e. being applied a voltage in order to transmit a pressure signal, or receiving a pressure signal and emitting an electric signal, it is obvious that instead of the two transducers 3, 5 in Fig. 1, it is in principle possible to use one single transducer, in which case the signal source 2 should be designed electronically in such a way that, when operated from the analysis unit 4 and the computer 6, it firstly emits a transient signal and subsequently transfers the response signal to the analysis unit. If a random or a pseudo-random signal is used as an excitation signal, emitted continuously in the measuring period, two separate transducers will be used, though, as shown in Fig. 1.

It should be added as well that the invention also offers the possibility of prostate or uterus examination, etc.

Finally, it should be mentioned that the invention also offers the possibility for reflectometric examination of other cavities, e.g. continuous monitoring of the cavity in an item manufactured by an extrusion process, as the technique of the invention allows for closely monitoring the extrusion parameters in order to e.g. obtain a constant wall thickness of the item, which could e.g. be a hose.

## Claims

1. A hose for reflectometric examination and measurement of cavities by means of acoustic reflectometry, having a distal end intended to be introduced via an entrance to a cavity, **characterised in:**
- **that** the hose is at least in a measuring zone designed with a thin outer wall made from a soft and/or elastic plastics or elastomeric material, whereby position or movement of the walls of the cavity will be transferred into corresponding position or movement of the thin outer wall of the hose, and
- **that** the measuring zone is positioned at or at a distance from the distal end of the hose.

2. A hose according to claim 1, **characterised in that** the hose (1) has one longitudinal, axially centred lumen (19).

3. A hose according to claim 1, **characterised in that** the hose (1) has two longitudinal lumens separated from each other by a diametric partition.

4. A hose according to claim 1, **characterised in that** the hose (10) has a longitudinal, axially centred lumen (11) surrounded by a ring-shaped wall (15) and a number of longitudinal canals (12, 13, 14), spaced around the ring-shaped wall (15) and separated from each other by means of essentially radial partitions (16, 17, 18).

5. A hose according to claim 4, **characterised in that** the hose has three longitudinal canals (12, 13, 14), said canals essentially being circular-arc shaped, seen in a sectional plane orthogonal to the longitudinal axis of the hose (1), and that there are essentially equal angular distances between the partitions (16, 17, 18).

6. A hose according to any of the preceding claims, **characterised in that** the proximal end (A) of the hose (1) is adapted for connection to auxiliary means for introducing the hose (1) forward to the cavity in question.

7. A hose according to any of the preceding claims, **characterised in that** the hose (1) at its proximal end has means so as to pressurise the lumen and/or lumens and/or each of the chambers with a positive or negative pressure relative to the environment.

8. A hose according to any of the preceding claims, **characterised in that** the proximal end of the hose (1) is adapted for attaching a transducer for transfer of an acoustic signal to the lumen of the hose.

9. A hose according to any of the preceding claims, **characterised in that** the hose has over at least part of its longitudinal extension, and preferably at its distal end, at least one zone (B) with an increased outer wall flexibility.

10. A hose according to claim 9, **characterised in that** said zone (B) with increased outer wall flexibility is created by the outer wall locally having a reduced wall thickness.

11. A hose according to claim 9, **characterised in that** said zone (B) with increased outer wall flexibility is created by a local change in the quality of the hose outer wall material.

12. A hose according to any of the preceding claims, **characterised in that** the hose further has a longitudinal canal for blood circulation from blood admission openings (33) at the distal end of the hose to a blood outlet at a distance from the distal end.

13. A hose according to any of the claims 1-11 and for examination of the air passages, **characterised in that** the hose further has a longitudinal canal for venting the air passages during the examination performed.

14. A hose according to any of the preceding claims, **characterised in that** the hose is closed at its distal end.

15. A device for examination and measurement of cavities by means of acoustic reflectometry, the device comprising an electronic signal source; a hose with a distal end (B) intended to be introduced via an entrance to a cavity, a bi-directional electro-acoustic transducer for transferring an activating signal from the signal source to the hose and forward through same and for receiving response signals from the hose, the transducer being connected to the hose near the proximal end of same, and a computer with a signal-analysis processor adapted for analysis of the response signals in relation to the activation signal, **characterised in:**
- **that** the hose is at least in a measuring zone designed with a thin outer wall made from a soft and/or elastic plastics or elastomeric material, whereby position or movement of the walls of the cavity will be transferred into corresponding position or movement of the thin outer wall of the hose, and
- **that** the measuring zone is positioned at or at a distance from the distal end of the hose.

16. A device according to claim 15, **characterised in that** the hose (1) has one longitudinal, axially centred lumen (19).

17. A device according to claim 15 or 16, **characterised in that** the proximal end (A) of the hose (1) is adapted for connection to auxiliary equipment for introducing the hose (1) forward to the cavity in question.

18. A device according to any of the claims 15-17, **characterised in that** the hose (1) at its proximal end has means so as to pressurise the lumen with a positive or negative pressure relative to the environment.

19. A device according to any of the claims 15-18, **characterised in that** the proximal end of the hose (1) is adapted for attaching said transducer for transfer of an activation signal from said signal source to the lumen.

20. A device according to any of the claims 15-19, **characterised in that** the hose (1) has over at least part of its longitudinal extension, and preferably at its distal end, at least one zone (B) with an increased outer wall flexibility.

21. A device according to claim 20, **characterised in that** said zone (B) with increased outer wall flexibility is created by the outer wall locally having a reduced wall thickness.

22. A device according to claim 20, **characterised in that** said zone (B) with increased outer wall flexibility is created by a local change in the quality of the hose outer wall material.

23. A device according to any of the claims 15-22, **characterised in that** the hose is closed at its distal end.
